# EUROPEAN PATENT APPLICATION

(11) **EP 2 486 849 A2**
(43) Date of publication of application: **15.08.2012**
(21) Application number: 12151382.4
(22) Date of filing: 15.05.2008
(51) Int. Cl.: A61B 5/055, G01R 33/563, G01R 33/56, A61K 49/00

(54) **Assessment of blood-brain barrier disruption**

(30) Priority: 16.05.2007 US 924474 P
(62) Divisional of application: 08751361.0
(71) Applicant: Yeda Research and Development Co. Ltd., 76100 Rehovot (IL); Tel HaShomer Medical Research Infrastructure and Services Ltd., 52 621 Ramat Gan (IL)
(72) Inventor: Israeli, David, 76100 Rehovot (IL); Mardor, Yael, 42655 Natania (IL); Volk, Talila, 76100 Rehovot (IL)
(74) Representative: Fichter, Robert Arno

(57) **Abstract**

The present invention relates to a system for disrupting blood-brain barrier of a subject, comprising a transcranial magnetic stimulation device having a mode of operation which effects a blood-brain barrier disruption.

## Description

### FIELD AND BACKGROUND OF THE INVENTION

The present invention, in some embodiments thereof, relates to medicine and, more particularly, but not exclusively, to assessment of blood brain barrier disruption via magnetic resonance imaging.

Blood-Brain Barrier (BBB) is a capillary barrier comprising a continuous layer of tightly bound endothelial cells. These endothelial cells are different from those found in other tissues of the body. In particular, they form complex tight junctions between themselves. The actual BBB is formed by these tight intercellular junctions which, together with the cells themselves, form a continuous wall against the passive movement of many molecules from the blood to the brain. These cells are also different in that they have few pinocytotic vesicles, which in other tissues allow somewhat unselective transport across the capillary wall. In addition, continuous gaps or channels running through the cells, which would allow unrestrained passage, are absent.

One function of the BBB is to protect the brain from fluctuations in blood chemistry. However, this isolation of the brain from the bloodstream is not complete, since an exchange of nutrients and waste products does exist. The presence of specific transport systems within the capillary endothelial cells assures that the brain receives, in a controlled manner, all of the compounds required for normal growth and function.

The obstacle presented by the BBB is that, in the process of protecting the brain, it excludes many potentially useful therapeutic and diagnostic agents. Administration of therapeutic agents for the treatment of central nervous system (CNS) pathologies is thus mostly inefficient due to poor penetration of most drugs across the BBB.

The unique biological aspect of the BBB is oftentimes addressed in the context of treatment of central nervous system (CNS) disorders. While the interendothelial junctions between the cells of the BBB are normally designed to keep potentially noxious substances away from the brain, this condition may change for patients suffering from a CNS disorder or having brain abscesses, inflammation or tumors. For example, it has been repotted that patients suffering from multiple sclerosis,

Alzheimer's, stroke and brain trauma experience breakdown of BBB (see, *e.g*., Ballabh, et al. (2004), "The blood-brain barrier: an overview: structure, regulation, and clinical implications," Neurobiol Dis 16(1):1-13].

Over the years, extensive research has been made in connection to BBB. Attempts have made to develop agents capable of crossing the BBB (see, *e.g*., U.S. Patent Nos. 4,801,575, 5,004,697, 6,419,949 and 6,294,520), agents which increase BBB permeability (see, *e.g*., U.S. Patent Nos. 5,434,137, 5,506,206 and 5,591,715), and various techniques for delivering substances across the BBB (see, *e.g*., U.S. Patent Nos. 5,670,477, 5,752,515 and 6,703,381), treating a damaged BBB (see, *e.g*., U.S. Patent No. 4,439,451), analyzing the BBB (see, *e.g*., U.S. Patent No. 6,574,501 and Wang el al., 2006, "Vascular Volume and Blood-Brain Barrier Permeability Measured by Dynamic Contrast Enhanced MRI in Hippocampus and Cerebellum of Patients with MCI and Normal Controls," J Magn Reson Imaging 24:695-700), and the like.

Numerous attempts have also been made to develop techniques for testing the ability of substances to cross the BBB. To this end see, *e.g*., U.S. Patent No. 5,266,480; Latour et al. (2004), "Early blood-brain barrier disruption in human focal brain ischemia," Ann Neurol 56(4):468-77; Ewing et al. (2003), "Patlak plots of Gd-DTPA MRI data yield blood-brain transfer constants concordant with those of 14C-sucrose in areas of blood-brain opening," Magn Reson Med 50(2):283-92; Taheri, S. and R. Sood (2006), "Kalman filtering for reliable estimation of BBB permeability," Magn Reson Imaging 24(8):1039-49; and Tomkins et al. (2007), "Blood-Brain Barrier Disruption in Post-Traumatic Epilepsy," J Neurol Neurosurg Psychiatry.

### SUMMARY OF THE INVENTION

According to an aspect of some embodiments of the present invention there is provided a method of analyzing a blood-brain barrier of a subject having therein a detectable dose of an MRI contrast agent. The method comprises: acquiring a plurality of magnetic resonance images of the subject's brain over a predetermined time-period; comparing at least two of the plurality of magnetic resonance images thereamongst so as to determine variations in concentration of the contrast agent in the brain; assessing blood-brain barrier function based on the variations; and issuing a report regarding the blood-brain barrier function.

According to some embodiments of the invention the method further comprises mapping the concentration variations, wherein the report comprises a blood-brain barrier function map.

According to some embodiments of the invention the comparison comprises constructing a plurality of normalized intensity maps each being associated with one magnetic resonance images, wherein the mapping of the concentration variations comprises detecting dissimilarities among a pair of intensity maps so as to construct at least one variation map describing the concentration variations.

According to some embodiments of the invention the determination of the variations comprises assigning a representative intensity value for one or more regions of interest within the magnetic resonance image and determining a time-dependence of the representative intensity value.

According to some embodiments of the invention the method further comprising generating a graph describing the time-dependence.

According to an aspect of some embodiments of the present invention there is provided a method of determining the effect of a compound on a blood-brain barrier of a subject, comprising administering the compound and a detectable dose of MRI contrast agent and executing the method described above.

According to an aspect of some embodiments of the present invention there is provided a method of monitoring BBB function at the time of delivery of a compound to the brain, comprising administering the compound and a detectable dose of MRI contrast agent and executing the method described above, thereby monitoring BBB function at the time of the delivery.

According to some embodiments of the invention the method further comprising administrating a blood-brain barrier modifying agent capable of temporarily generating blood-brain barrier disruption.

According to some embodiments of the invention the blood-brain barrier modifying agent comprises Isosorbide dinitrate. According to some embodiments of the invention the blood-brain barrier modifying agent comprises Hydroxizine. According to some embodiments of the invention the blood-brain barrier modifying agent comprises an anti histamine. According to some embodiments of the invention the blood-brain barrier modifying agent is capable of modifying serotonin levels. According to some embodiments of the invention the blood-brain barrier modifying agent is an antipsychotic agent. According to some embodiments of the invention the blood-brain barrier modifying agent comprises an glutamate receptor agonist or an antagonist. According to some embodiments of the invention the blood-brain barrier modifying agent is an anti-inflammatory agent. According to some embodiments of the invention the blood-brain barrier modifying agent is an anti-hypertensive agent. According to some embodiments of the invention the blood-brain barrier modifying agent comprises a central nervous system stimulant.

According to an aspect of some embodiments of the present invention there is provided a method of preventing or reducing disruption of blood-brain barrier of a subject during treatment. The method comprises: administering a detectable dose of MRI contrast agent to the subject; executing the method described above; and generating a detectable signal when a predetermined criterion pertaining to blood-brain barrier dysfunction is met, thereby preventing or reducing the disruption of the blood-brain barrier.

According to an aspect of some embodiments of the present invention there is provided a method of detecting a central nervous system disorder. The method comprises executing the method described above so as to determine blood-brain barrier dysfunction thereby detecting the central nervous system disorder.

According to some embodiments of the invention the method further comprises staging the central nervous system disorder based on the blood-brain barrier dysfunction.

According to some embodiments of the invention the central nervous system disorder is Schizophrenia. According to some embodiments of the invention the central nervous system disorder is a migraine or headache disorder. According to some embodiments of the invention the central nervous system disorder is Parkinson.

According to an aspect of some embodiments of the present invention there is provided apparatus for analyzing a blood-brain barrier of a subject from a plurality of magnetic resonance images of the subject's brain acquired over a predetermined time-period. The subject having therein a detectable dose of an MRI contrast agent. The apparatus comprises: an intensity map constructor for constructing, for each magnetic resonance image, an intensity map; a variation map constructor for constructing at least one variation map describing variations in concentration of the contrast agent in the brain by detecting dissimilarities among a pair of intensity maps; and blood-brain barrier function assessment unit configured for assessing blood-brain barrier function based on the variations and for issuing a report regarding the blood-brain barrier function.

According to some embodiment of the invention the assessment unit is configured for assigning a representative intensity value for a region-of-interest within the magnetic resonance image and determining a time-dependence of the representative intensity value.

According to some embodiments of the invention the assessment unit is configured for generating a graph describing the time-dependence.

According to some embodiments of the invention each representative intensity value is assigned by averaging intensities over a respective magnetic resonance image.

According to some embodiments of the invention the subject is immobilized while the magnetic resonance images are acquired.

According to some embodiments of the invention each magnetic resonance image comprises a sliced magnetic resonance image, wherein the comparison is performed slice by slice.

According to some embodiments of the invention the variation map(s) comprises a subtraction map.

According to some embodiments of the invention variation map(s) comprises a slope map.

According to some embodiments of the invention variation map(s) comprises a ratio map.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

Implementation of the method and/or system of embodiments of the invention can involve performing or completing selected tasks manually, automatically, or a combination thereof. Moreover, according to actual instrumentation and equipment of embodiments of the method and/or system of the invention, several selected tasks could be implemented by hardware, by software or by firmware or by a combination thereof using an operating system.

For example, hardware for performing selected tasks according to embodiments of the invention could be implemented as a chip or a circuit. As software, selected tasks according to embodiments of the invention could be implemented as a plurality of software instructions being executed by a computer using any suitable operating system. In an exemplary embodiment of the invention, one or more tasks according to exemplary embodiments of method and/or system as described herein are performed by a data processor, such as a computing platform for executing a plurality of instructions. Optionally, the data processor includes a volatile memory for storing instructions and/or data and/or a non-volatile storage, for example, a magnetic hard-disk and/or removable media, for storing instructions and/or data. Optionally, a network connection is provided as well. A display and/or a user input device such as a keyboard or mouse are optionally provided as well.

### BRIEF DESCRIPTION OF THE DRAWING

Some embodiments of the invention are herein described, by way of example only, with reference to the accompanying drawings and images. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.

In the drawings:
FIG. 1 is a flowchart diagram describing a method suitable of analyzing a blood-brain barrier of a subject, according to some embodiments of the present invention;
FIG. 2 is a flowchart diagram of a comparison procedure according to some embodiments of the present invention;
FIG. 3 is a schematic illustration of apparatus for analyzing a blood-brain barrier of a subject, according to some embodiments of the present invention;
FIG. 4 is a schematic illustration of a magnetic resonance imaging system for imaging a body, according to some embodiments of the present invention;
FIGs. 5a-b show intensity maps (Figure 5a) and an intensity plot (Figure 5b) of a mouse that died during an experiment performed according to some embodiments of the present invention;
FIG. 5c shows an intensity plot of a mouse which was kept alive throughout an experiment performed according to some embodiments of the present invention;
FIGs. 6a-d show intensity plots (average normalized intensity in dimensionless units as a function of time in minutes) of a control rat (Figure 6a-b) and a rat treated with SNP (Figure 6c-d), as obtained during an experiment performed according to some embodiments of the present invention;
FIGs. 7a-f are subtraction maps of a treated rat (Figures 7a-c) and a control rat (Figures 7d-f), as obtained during an experiment performed according to some embodiments of the present invention;
FIG. 8 is a graph showing the average subtraction values of treated rats (blue diamonds) and control rats (pink squares) as obtained during an experiment performed according to some embodiments of the present invention;
FIGs. 9a-d are fluorescence images of two treated rats (Figures 9a-b) and two control rats (Figures 9c-d) as obtained during an experiment performed according to some embodiments of the present invention;
FIGs. 10a-b are T1-weighted MR images acquired during an experiment performed according to some embodiments of the present invention from the healthy subject 1 minute (Figure 10a) and 10 minutes (Figure 10-b) after injection of a contrast agent;
FIG. 10c is a subtraction map corresponding to the MR image shown in Figure 10b;
FIGs. 11a-e are T₁-weighted MR images acquired during an experiment performed according to some embodiments of the present invention from the schizophrenia patient during acute psychotic state 1, 7, 13, 19 and 23 minutes after injection of contrast agent;
FIG. 12a-e are intensity maps which respectively correspond to the MR image shown in Figures 11a-e;
FIG. 12f shows a color scale for Figures 12a-e;
FIGs. 13a-d are subtraction maps which respectively correspond to the intensity maps shown in Figures 12b-e;
FIG. 13e shows a color scale for Figures 13a-d;
FIGs. 14a-b are T₁-weighted MR images acquired during an experiment performed according to some embodiments of the present invention from a subject suffering from meningioma, 1 minute (Figure 14a) and 7 minutes (Figure 14b) after injection of contrast agent;
FIG. 14c is a subtraction map corresponding to the MR images shown in Figure 14a-b;
FIGs. 15a-b are T₁-weighted MR images acquired during an experiment performed according to some embodiments of the present invention from a subject suffering from cappilay angioma, 1 minute (Figure 15a) and 10 minutes (Figure 15b) after injection of contrast agent; and
FIGs. 15c-d are a subtraction map (Figure 15c) and a ratio map (Figure 15d) corresponding to the MR images shown in Figure 15a-b.

### DESCRIPTION OF SPECIFIC EMBODIMENTS OF THE INVENTION

The present invention, in some embodiments thereof, relates to medicine and, more particularly, but not exclusively, to assessment of blood brain barrier disruption via magnetic resonance imaging (MRI).

Before explaining at least one embodiment of the invention in detail, it is to be understood that the invention is not necessarily limited in its application to the details of construction and the arrangement of the components and/or methods set forth in the following description and/or illustrated in the drawings and/or the Examples. The invention is capable of other embodiments or of being practiced or carried out in various ways.

MRI is a method to obtain an image representing the chemical and physical microscopic properties of materials, by utilizing a quantum mechanical phenomenon, named Nuclear Magnetic Resonance (NMR), in which a system of spins, placed in a magnetic field resonantly absorb energy, when applied with a certain frequency.

When placed in a magnetic field, a nucleus having a spin I is allowed to be in a discrete set of energy levels, the number of which is determined by I, and the separation of which is determined by the gyromagnetic ratio g of the nucleus and by the magnetic field. Under the influence of a small perturbation, manifested as a radiofrequency magnetic field rotating about the direction of a primary static magnetic field, the nucleus has a time-dependent probability to experience a transition from one energy level to another. With a specific frequency of the rotating magnetic field, the transition probability may reach the value of unity. Hence, at certain times a transition is forced on the nucleus, even though the rotating magnetic field may be of small magnitude relative to the primary magnetic field. For an ensemble of spin I nuclei, the transitions are realized through a change in the overall magnetization.

Once a change in the magnetization occurs, a system of spins tends to restore its magnetization longitudinal equilibrium value, in accordance with the thermodynamic principle of minimal energy. The time constant which control the elapsed time for the system to return to the equilibrium value is called "spin-lattice relaxation time" and is denoted T₁. An additional time constant, T₂ (≤T₁), called "spin-spin relaxation time", controls the elapsed time in which the transverse magnetization diminishes, in accordance with the thermodynamic principle of minimal energy. However, inter-molecule interactions and local variations in the value of the static magnetic field alter the value of T₂ to an actual value denoted T₂*.

In MRI, a static magnetic field having a predetermined gradient is applied on an object, thereby creating, at each region of the object, a unique magnetic field. By detecting the NMR signal, knowing the magnetic field gradient, the position of each region of the object can be imaged.

Magnetic resonance (MR) pulse sequences applied to the object (*e.g*., a patient) induce, refocus and/or manipulate the magnetic resonance by interacting with the spins. NMR signals are generated and used for obtaining information and reconstruct images of the object. The above mentioned relaxation times and the density distribution of the nuclear spins are properties which vary from one normal tissue to the other, and from one diseased tissue to the other. These quantities are therefore responsible for contrast between tissues in various imaging techniques, hence permitting image segmentation.

Many MR sequences are known. Broadly speaking, the various time instants of the MR sequences are selected so as to encode the magnetic resonance to provide spatial information, flow information, diffusion information and the like.

In diffusion-weighted MRI, for example, the magnetic field gradients are selected so as to provide motion-related contrast which is sensitive to motion of fluid molecules in selected directions. Diffusion-weighted MRI exploits the random motion of the molecules which causes a phase dispersion of the spins with a resultant signal loss.

In T₂-weighted MRI, the MR sequence is selected so as to control the T₂ relaxation process, and minimize T1 effect. One method for such control is called the spin-echo method, in which the magnetization is first forced to lie in the transverse plane and, after a predetermined time-interval, refocused by a 180° flip. The peaks of the resulting signal are described by a decay curve characterized by the T₂ time-constant.

The present embodiments exploit the advantages of MRI for assessment of BBB disruption.

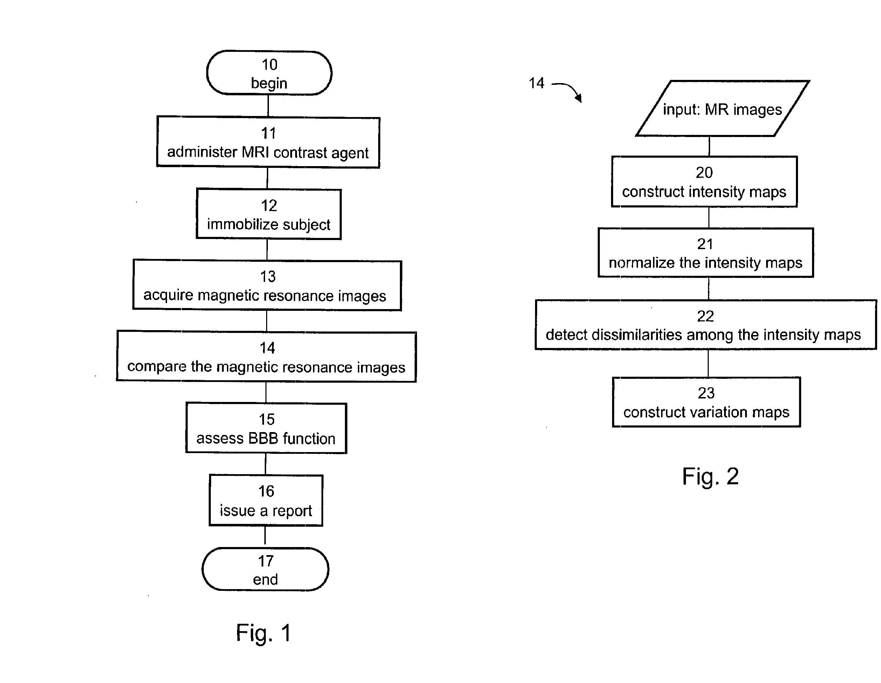

Referring now to the drawings, Figure 1 is a flowchart diagram describing a method suitable of analyzing a blood-brain barrier of a subject.

The method begins at **10** and optionally continues to **11** which describers a process in which a detectable dose of-an MRI contrast agent is administered to the subject. Alternatively the method can begin while the subject already has the contrast agent in his or her vasculature.

The term "detectable dose" refers to a dose which allows detection of the contrast agent in an MRI system. For example, when the MRI contrast agent is Gd-DTPA, a detectable dose can be from about 0.2 ml/kg to about 0.6 ml/kg. However, this need not necessarily be the case, since, for some embodiments, another type of contrast agent and/or another dose can be utilized.

The MRI contrast agent can be either a positive or a negative MRI contract agent. A positive MRI contract agent is an agent which increases the NMR signal relative to nearby tissues or fluids, and a negative MRI contract agent is an agent which decreases the NMR signal relative to the nearby tissues of fluids. In any event, the MRI contrast agent is detectable since it is distinguished from its surroundings either by an enhanced or reduced NMR signal.

In various exemplary embodiments of the invention a positive MRI contrast agent is used such that its dominant effect is to reduce the T₁ relaxation time. In some embodiments the MRI contrast agent reduces the T₂ relaxation time.

The magnetic properties of the MRI contrast agent can be of any type. More specifically, the MRI contrast agent comprises a magnetic material which can be paramagnetic, superparamagnetic or ferromagnetic material.

The magnetic properties of the MRI contrast agent (and all other materials in nature) originate from the sub-atomic structure of the material. The direction as well as the magnitude of the magnetic force acting on the material when placed in a magnetic field is different for different materials. Whereas the direction of the force depends only on the internal structure of the material, the magnitude depends both on the internal structure as well as on the size (mass) of the material. Ferromagnetic materials have the largest magnetic susceptibility compared to para- or superparamagnetic materials. Superparamagnetic materials consist of individual domains of elements that have ferromagnetic properties in bulk. Their magnetic susceptibility is larger than that of the paramagnetic but smaller than that of ferromagnetic materials.

Broadly speaking, ferromagnetic and superparamagnetic MRI contrast agents are negative MRI contrast agents and paramagnetic MRI contrast agents can be either negative or positive MRI contrast agents. The effect of paramagnetic material on the magnetic resonance signal dependents on the type and concentration of the paramagnetic material, as well as on external factors, such as the strength of the applied magnetic field. In various exemplary embodiments of the invention the MRI contrast agents which comprise paramagnetic materials are positive contrast agents.

Paramagnetic materials, as used herein, refers to metal atoms or ions which are paramagnetic by virtue of one or more unpaired electrons, and excludes radioactive metal atoms or ions commonly referred to as radionuclides. Representative examples include, without limitation, the paramagnetic transition metals and lanthanides of groups 1b, 2b, 3a, 3b, 4a, 4b, 5b, 6b, 7b, and 8, more preferably those of atomic number 21-31, 39-50, 57-71, and 72-82, yet more preferably gadolinium (Gd), dysprosium (Dy), chromium (Cr), iron (Fe), and manganese (Mn), still more preferably Gd, Mn, and Fe, and most preferably Gd.

The use of Gd-based contrast agents are particularly advantageous because they are generally accessible, approved for safely and not expensive. Such contrast agents depict clearly on T₁-weighted MRI and can be found in different molecular size for depicting different aspects of BBB functioning

In various exemplary embodiments of the invention the MRI contrast agent comprises a chelating moiety, capable of forming chelate-complexes with the magnetic material. These can be linear chelating moieties such as, but not limited to, polyamino polyethylene polyacetic acids [*e.g*., diethylenetriamine pentaacetic acid (DTPA), ethylene diamine tetraacetic acid (EDTA), triethylene tetraamine hexaacetic acid (TTHA) and tetraethylene pentaamine heptaacetic acid]; or cyclic chelating moieties such as, but not limited to, polyazamacrocyctic compounds [*e.g*., such as 1,4,7,10-tetra-azacyclododecane-1,4,7,10-tetraacetic acid (DOTA)].

The use of DTPA is particularly advantageous because it is a small and stable molecule, which is generally accessible.

In various exemplary embodiments of the invention the MRI contrast agent is a positive MRI contrast agent which comprises Gd-DTPA. Gd-DTPA is a positive contrast agent when observed via T₁-weighted MRI and a negative contrast agent when observed via T₂-weighted MRI. As T₁ is more sensitive to Gd-DTPA, T₁-weighted MRI is the preferred MRI technique when the contrast agent is Gd-DTPA.

Referring again to Figure 1, at 13 a plurality of MR images of the subject's brain are acquired over a predetermined time-period. The MR images are preferably acquired substantially continuously or at least repeatedly over the time-period. Typically, but not obligatorily, the time-period is sufficiently long so as to allow assessment of early as well as late BBB disruption. In various exemplary embodiments of the invention the MR images are acquired over a period of at least 10 minutes, more preferably at least 20 minutes, more preferably at least 30 minutes, *e.g*., about 35 minutes or about 40 minutes or more.

The acquisition of MR images can include a slicing technique, in which case one or more of the MR images (*e.g*., each MR image) is a sliced MR image which comprises a set of MR images, wherein each element in the set corresponds to a different slice of the brain. The thickness of each slice can be selected to improve the signal-to-noise ratio (SNR) and/or the contrast-to-noise ratio (CNR) of the image. Typically, but not obligatorily, there are about 20-25 image slices in a set.

The acquisition time of a set of slices generally depends on the pulse sequence which is employed. A typical acquisition time suitable for the present embodiments, is, without limitation, from about 1 minute to about 5 minutes. Thus, when the method acquires MR images over a period of, say, 40 minutes, the number of sets is from about 8 sets to about 40 sets. In some embodiments of the invention T₁-weighted fast spin-echo MR images are acquired with an acquisition time of about 2 minutes per set.

Beside the subject's brain, a phantom sample can also be scanned by MRI for calibration purposes. The phantom sample is preferably made of a material suitable for MRI with relaxation times T₁ and T₂ which are similar to those of human tissue for the particular MRI system used. For example, the phantom can be a tube filled with soap water, carrageenan gel or the like. The phantom sample can be placed near the head of the subject such that during acquisition, NMR signals are collected from both the brain and the phantom.

In various exemplary embodiments of the invention the acquisition of MR images is preceded by a procedure in which the subject is immobilized (see 12). This can be done physically, *e.g*., by means of a holding device such as a head immobilizer, and/or chemically *e.g*., by means of sedation or general anesthesia. This embodiment is particularly useful when the subject suffers from a CNS disorder which prevents him or her from lying still. In this case immobilization facilitates better quality of MR images and allows comparison among the acquired MR images since the position of the subject with respect to the MRI system does not vary with time.

At **14,** at least a few of the MR images are compared thereamongst, so as to determine variations in concentration of the contrast agent in the brain. In various exemplary embodiments of the invention the comparison is performed in pairs, whereby each time two MR images are compared. When the images are sets of slices the comparison is preferably performed slice-by-slice. A comparison procedure according to some embodiments of the present invention is provided hereinafter with reference to Figure 2.

At **15,** the method assesses the BBB function based on the variations in contrast agent concentration. Specifically, when the concentration of contrast agent in the brain tissue increases with time, the method can identify BBB disruption. The assessment can be done globally and/or locally.

In global assessment, the method determine whether or not there is an increment in the overall amount of contrast agent in the brain, whereby such increment as a function of time can be identified as BBB disruption. This can be done by assigning a representative intensity value for each MR image (or each set of MR images) of the sequence and determining the time-dependence of the representative intensity value over the sequence. The representative intensity value can be calculated by integrating or averaging the intensities over the image or set of images. The integration or averaging can also be weighted according to some predetermined weighting scheme. When the representative intensity value is obtained by averaging, any averaging technique can be employed, including, without limitation, arithmetic mean, center-of-mass and the like.

The assigned representative intensity values and optionally their time-dependence can be stored in a computer memory medium. The representative intensity values can also be visualized, *e.g*., by constructing a graph of the representative intensity value as a function of the time. An example of such graph is provided in the Examples section that follows. The time-dependence of the representative intensity value can be used for assessing the BBB function whereby, for example, an increment of the representative intensity value with time can indicate BBB disruption and constant or decrement can indicate intact BBB.

In local assessment, the method determines the location in the brain at which there is an increment of contrast agent concentration. For example, the method can map the concentration variations over the brain or a region-of-interest therein, as further detailed hereinunder.

At **16** the method issues a report regarding the BBB function. The report can include indication whether or not a BBB disruption has been identified and/or indication regarding the extent of BBB disruption (*e.g*., rate of BBB crossing for a given compound). The report can be global in the sense that it provides indication regarding BBB disruption for the entire brain or region-of-interest therein and/or local in the sense that it may also include information regarding the localization of BBB disruption. For example, the report can be a BBB function map which describes the BBB function or BBB dysfunction for a plurality of locations over the brain or a region-of-interest therein.

The method ends at **17.**

Reference is now made to Figure 2 which is a flowchart diagram of a comparison procedure according to some embodiments of the present invention. The procedure can be employed by the method described in the flowchart diagram of Figure 1 (see **14**).

The input data to the comparing procedure include a plurality of MR images or a plurality of sets of MR images as further detailed hereinabove (see 13). The MR images or sets of MR images are time-ordered, thus forming a sequence of MR images or a sequence of sets of MR images.

At **20,** the procedure constructs a plurality of intensity maps. Each intensity map is associated with one MR image or one set of MR images. The intensity map includes intensity values for a plurality of locations (*e.g*., pixels) over the image or a region-of-interest therein. When the intensity map is associated with a set of images, the intensity values can be obtained by averaging over the set. Any averaging technique can be employed, including, without limitation, arithmetic mean, center-of mass, and the like. The averaging is preferably performed location-wise. For example, the *i*th intensity value of a particular intensity map can be obtained by averaging intensities as obtained from the *i*th location of the first slice, the ith location of the second slice and so on. The averaging can also be over a specific brain organelle or over white matter or gray matter which can be determined, *e.g*., by segmentation methods.

Since each intensity map is associated with one MR image (or one set of MR images), the intensity maps also form a time-ordered sequence. The intensity maps sequence is preferably stored in a computer memory for further processing. One or more of the intensity maps can also be visualized, *e.g*., on a display device.

In various exemplary embodiments of the invention the intensity maps are normalized (see 21). The normalization is typically with respect to a reference intensity value which remains substantially constant over the sequence. Such reference intensity value can be obtained, for example, from a phantom sample which can be scanned by MRI together with the brain. During normalization of an intensity map, each intensity value of the map is divided by the reference intensity value to provide a normalized intensity value.

At 22 the procedure detects dissimilarities among two or more of the intensity maps. When the aforementioned normalization is employed, the procedure detects the dissimilarities after normalization. In some embodiments, dissimilarities are detected pairwise. In these embodiments, dissimilarities are detected between the *n*th intensity map and the *m*th intensity map, where m and *n* (*m* ≠ *n*) are positive integers representing the position of the respective intensity map within the time-ordered sequence. In various exemplary embodiments of the invention *n* = 1 and *m* > 1. In other words, in these embodiments dissimilarities are detected with respect to the first intensity map (associated with the first MR image or the first set of MR images which was acquired after contrast agent administration). Thus, the procedure can detect dissimilarities between the intensity values of the first and second intensity map, then between the intensity values of the first and third intensity maps and so on. Detection of dissimilarities among other pairs of intensity maps (*m*, *n ≠* 1) is also contemplated, particularly, but not obligatorily, when identification of late BBB disruptions is of interest.

Dissimilarities can be detected by subtraction, division or combination thereof. Thus, when the procedure detect, *e.g*., dissimilarities between the first and second intensity maps, the procedure can subtract the intensity values of the first intensity map from the respective intensity values of the second intensity map to provide a subtraction value, or the procedure can divide the intensity values of the second intensity map by the respective intensity values of the first intensity map to provide a ratio value. The procedure can also obtain a slope value, by dividing the subtraction value or ratio value by the time difference between the two maps. Dissimilarities can also be detected using other operations such as subtraction of logarithms and the like.

At **23** the procedure constructs one or more variation maps using the detected dissimilarities. Each variation map preferably describes dissimilarities among a pair of intensity maps and includes variation values which respectively correspond to locations over the image. The number of variation maps is typically at least *N*-1, where *N* is the number of intensity maps. The number of variation maps can be as large as the number of pairs in the sequence of intensity maps. The variation values of a variation map can be, for example, subtraction values, in which case the map is referred to as a subtraction map, ratio values, in which case the map is referred to as a ratio map, or slope values, in which case the map is referred to as a slope map.

The variation maps are preferably stored in a computer memory medium. One or more of the variation maps can also be visualized, *e.g*., on a display device. The variation values (subtraction, ratio, slope, *etc*) of the variation maps correspond to variations in the concentration of the MRI contrast agent in the brain. Thus, from the memory medium in which they are stored, the variation maps can be retrieved and searched so as to assess (see **15**) the BBB function at one or more locations over the maps. For example, in brain tissue, large variations can indicate BBB disruption and low or no variations can indicate intact BBB at the respective locations. In blood vessels or structures consisting of high blood volume, low or no variations are typically expected due to clearance of contrast agent from the blood. In the cerebrospinal fluid (CSF) large variations can indicate BBB disruption or blood-CSF barrier disruption.

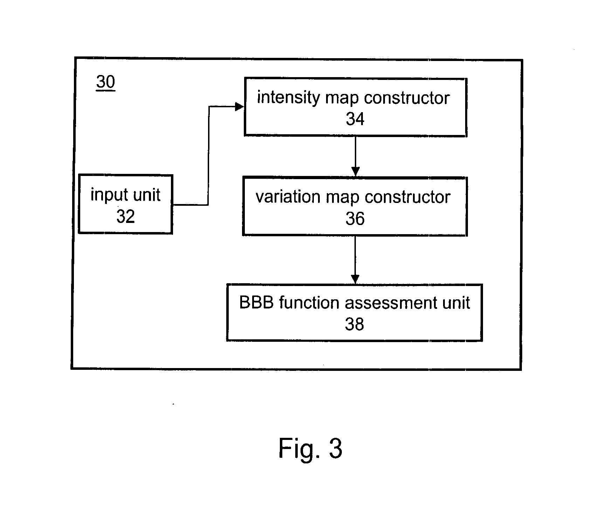

Reference is now made to Figure 3 which is a schematic illustration of an apparatus **30** for analyzing a blood-brain barrier of a subject, according to various exemplary embodiments of the present invention. Apparatus **30** can be utilized for executing selected steps of the method described above.

Apparatus **30** comprises an input unit **32** for inputting a plurality of MR images or a plurality of sereis of MR images as further detailed hereinabove. Apparatus 30 further comprises an intensity map constructor **34** for constructing, a plurality of intensity maps, each being associated with one MR image or one set of MR images, as further detailed hereinabove. Apparatus **30** further comprises a variation map constructor **36** for constructing one or more variation maps describing variations in concentration of the contrast agent in brain by detecting dissimilarities among a pair of intensity maps, as further detailed hereinabove. Apparatus **30** further comprises a BBB function assessment unit **38** configured for assessing BBB function based on the variations, as further detailed hereinabove. Unit **38** can issue a report regarding the BBB function.

In some embodiments, unit **38** assigns a representative intensity value for each MR image or set of MR images and determines the time-dependence of the representative intensity value as further detailed hereinabove. Unit **38** can also generate a graph describing the time-dependence.

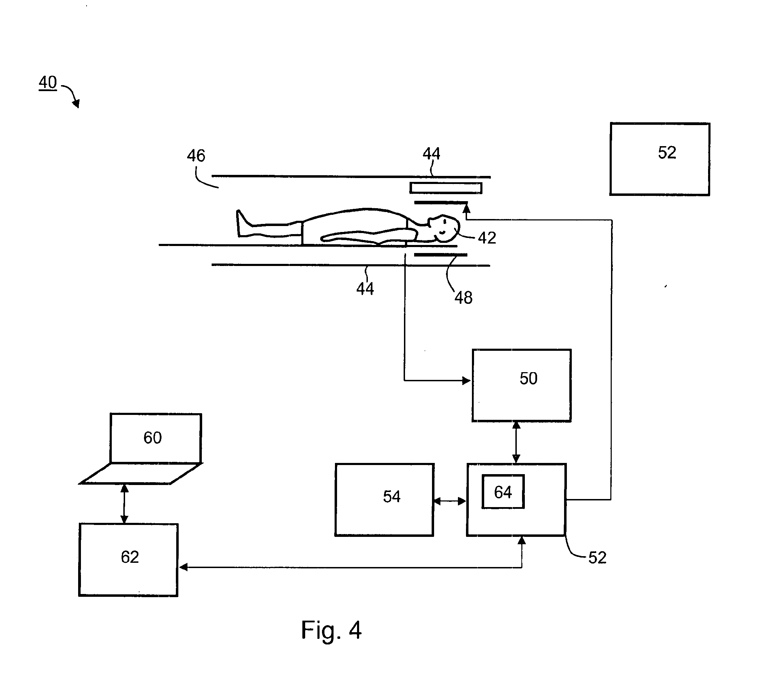

Reference is now made to Figure 4 which is a schematic illustration of a magnetic resonance imaging system **40** for imaging a brain **42,** according to various exemplary embodiments of the present invention. System **40** comprises a static magnet system **44** which generating a substantially homogeneous and stationary magnetic field B₀ in the longitudinal direction, a gradient assembly **46** which generates instantaneous magnetic field gradient pulses to form a non-uniform superimposed magnetic field, and a radiofrequency transmitter system **48** which generates and transmits radiofrequency pulses to brain **42.**

System **40** further comprises an acquisition system **50** which acquires magnetic resonance signal from the brain, and a control system **52** which is configured for implementing various pulse sequences. Control system **52** is also configured to control acquisition system **50.**

In various exemplary embodiments of the invention system **40** further comprises an image producing system **54** which produces magnetic resonance images from the signals of each acquisition. Image producing system **54** typically implements a Fourier transform so as to transform the data into an array of image data.

The operation of system **40** is preferably controlled from an operator console **60** which can include a keyboard, control panel a display, and the like. Console **60** can include or it can communicate with a data processor **62.** Data processor **62** may include apparatus **30,** and can therefore be used for analyzing BBB according to some embodiments of the present invention.

The gradient pulses and/or whole body pulses can be generated by a generator module **64** which is typically a part of control system **52.** Generator module **64** produces data which indicates the timing, strength and shape of the radiofrequency pulses which are to be produced, and the timing of and length of the data acquisition window.

Gradient assembly **46** typically comprises *Gₓ*, *G_{y}* and *G_{z}* coils each producing the magnetic field gradients used for position encoding acquired signals. Radiofrequency transmitter system **48** is typically a resonator which is used both for transmitting the radiofrequency signals and for sensing the resulting signals radiated by the excited nuclei in body **42.** The sensed magnetic resonance signals can be demodulated, filtered, digitized *etc.* in acquisition system **50** or control system **52.**

The method and apparatus of the present embodiments are useful for many medical applications.

In an aspect of some embodiments, a method for determining the effect of a compound on the BBB of the subject is provided. In this aspect the compound and a detectable dose of MRI contrast agent are administered to the subject, MR images are acquired and the BBB analysis method as described above is executed. The effect of the compound can be determined, for example, by comparing the BBB function assessment with and without compound administration. For example, if without compound administration the BBB is intact and after compound administration a BBB disruption is identified, the method can determine that the compound induces BBB disruption.

In an aspect of some embodiments, a method for monitoring BBB disruption during delivery of a compound, such as, but not limited to, a therapeutic pharmaceutical composition to the brain is provided. In this aspect, the compound and a detectable dose of MRI contrast agent are administered to the subject, and the BBB analysis method as described above is executed. The method can be preceded by administration of a BBB modifying agent which is capable of temporarily generating BBB disruption. Compound delivery can be controlled by monitoring BBB disruption prior to or during compound administration.

The BBB modifying agent can be an anti histamine, such as Hydroxyzine or the like. The BBB modifying agent can also affect the serotonin, for example, antidepressant (*e.g*., any type of serotonin specific reuptake inhibitors, including, without limitation, fluoxetine, Sertraline; any type of serotonin norepinehrine reuptake inhibitors; any type of monoamine oxidase inhibitor; and other antidepressants), antipsychotic (*e.g*., antipsychotics which have the ability to block serotonin receptor), and various agents for treating migraine (*e.g*., Triptans). The BBB modifying agent can be glutamate receptor agonist, antagonist or any other drug which affect the glutamate. Also contemplated are CNS stimulants (*e.g*., methylphenidate), alcohols, hallucinogens, opiates and inhalants and other psychotropic drugs that may have primary or secondary effect on biogenic amines and\or glutamate like anxiolitics, mood stabilizers, anticonvulsants, anesthetics and more. Additional compounds include anti inflammation drugs (*e.g*., steroids and non steroidal anti inflammatory drugs), anti hypertensive drugs (*e.g*., nitrates, beta blockers, ACE inhibitors), anti platlets drugs (*e.g*., aspirin), anticoagulants (*e.g*., warfarin) fibrinolytics (*e.g*., tissue plasminogen activator commonly known as tPA) and procoagulants (e.g hexakapron). Various BBB modifying agent are found in a review by Abbott et al., entitled "Astrocyte-endothelial interactions at the blood-brain barrier," published on January 2006 in Nature Reviews, Neuroscience 7:41-53.

In an aspect of some embodiments, a method for preventing or reducing BBB disruption in a subject during treatment is provided. The treatment can be any type of treatment which can potentially cause BBB disruption, including, without limitation, focused ultrasound/sound, radiofrequency treatment, laser and other thermal treatments, deep-brain stimulation, vagal brain stimulation, SPG stimulation, transcranial magnetic stimulation, electroconvulsive therapy, radiation and radiosurgery. In this aspect, a detectable dose of MRI contrast agent is administered to the subject, and the BBB analysis method as described above is executed. When a predetermined criterion pertaining to blood-brain barrier dysfunction is identified, the method can generate a detectable signal (*e.g*, alarm). Upon receipt of such signal, the treatment can be terminated, temporally ceased or modified, to prevent further BBB disruption.

The ability to assess BBB disruption during treatment is also useful for the development and safety approval of medical devices. For example, a device under development can be tested whether or not, or to what extent, it causes BBB disruption at a certain mode of operation. When BBB disruption is not desired, modes of operations at which there is a BBB disruption can be identified as less favored or harmful. When BBB disruption is desired, modes of operations can be categorized by their ability to modify the BBB. For example, a transcranial magnetic stimulation device or a high intensity ultrasound device can be tested to determine which mode of operation has minor or no affect on BBB. In such mode of operation a patient can be treated for a prolong period of time. Conversely, the device can be tested to determine which mode of operation causes BBB disruption. In such mode of operation a patient can be treated when it is desired to induce BBB disruption for short time-period (*e.g*., for the purpose of drug delivery).

The method of the present embodiments can also be used for monitoring BBB function while one or more of the above medical treatments is performed.

The method of the present embodiments can also be utilized for diagnosing a stroke or formulating a prognosis of a stroke. BBB opening is known to be a common side effect of stroke. When or after a patient experiences a stroke, the BBB analysis method as described above can be executed to determine whether or not the patient's BBB was disrupted, where it was disrupted and/or to what extent it was disrupted. Such determination may aid the physician in formulating prognosis and/or deciding on appropriate treatment. For example, it is known that treatment with Tissue Plasminogen Activator (tPA) may increase the risk of a hemorrhage. If the method of the present embodiments determines that the patient's BBB was not disrupted, the physician can determines that the patient is less likely to suffer from bleeding after tPA. Such information may increase the time window for treatment.

A typical infusion of tPA is over a period of 60 minutes or more starting with a bolus injection. According to some embodiments of the present invention the contrast agent can be injected at the time of bolus injection, and the BBB analysis method as described above can be executed. When BBB disruption is identified, the infusion of tPA can be terminated or titrated. Also contemplated is a procedure in which the BBB analysis method as described above is executed prior to the tPA treatment so as to assess BBB function, and if no BBB disruption is detected, tPA treatment can be initiated by bolus injection. The BBB analysis method can be continued during tPA infusion, so as to assess BBB function. When BBB disruption is identified, the infusion of tPA can be terminated or titrated.

In an aspect of some embodiments, a method for detecting a tumor in the brain is provided. In this aspect, a detectable dose of MRI contrast agent is administered to the subject, and the BBB analysis method as described above is executed. Upon detection of a local BBB disruption, the method can determine that it is likely that there is a tumor at the location of the BBB disruption. This aspect is particularly useful for tumors which are too small to be identified by conventional MRI. Thus, the present embodiments provide an early detection tool for brain tumors or a more accurate tool for determining the tumor borders.

In an aspect of some embodiments, a method for detecting a CNS disorder, such as, schizophrenia, Parkinson, migraine or headache disorder, is provided. In this aspect, a detectable dose of MRI contrast agent is administered to the subject, and the BBB analysis method as described above is executed. Upon detection of a certain pattern of BBB disruption, the method can access a database which includes BBB disruption pattern entries and a CNS disorder which corresponds to the pattern entries. If such entry exists in the database, the method can extract the corresponding CNS disorder and determine that it is likely that the subject suffers from this disorder.

In some embodiments of the invention the method is utilized for staging the CNS disorder. This can be done by determining the extent of BBB disruption or by analyzing modifications in the BBB disruption pattern.

As used herein the term "about" refers to ± 10 %.

The terms "comprises", "comprising", "includes", "including", "having" and their conjugates mean "including but not limited to".

The term "consisting of means "including and limited to".

The term "consisting essentially of" means that the composition, method or structure may include additional ingredients, steps and/or parts, but only if the additional ingredients, steps and/or parts do not materially alter the basic and novel characteristics of the claimed composition, method or structure.

As used herein, the singular form "a", "an" and "the" include plural references unless the context clearly dictates otherwise. For example, the term "a compound" or "at least one compound" may include a plurality of compounds, including mixtures thereof.

Throughout this application, various embodiments of this invention may be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

As used herein the term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

As used herein, the term "treating" includes abrogating, substantially inhibiting, slowing or reversing the progression of a condition, substantially ameliorating clinical or aesthetical symptoms of a condition or substantially preventing the appearance of clinical or aesthetical symptoms of a condition.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination or as suitable in any other described embodiment of the invention. Certain features described in the context of various embodiments are not to be considered essential features of those embodiments, unless the embodiment is inoperative without those elements.

Various embodiments and aspects of the present invention as delineated hereinabove and as claimed in the claims section below find experimental support in the following examples.

### EXAMPLES

Reference is now made to the following examples, which together with the above descriptions illustrate some embodiments of the invention in a non limiting fashion.

### EXAMPLE 1

### Animal Study

Following is a description of an animal study performed according to some embodiments of the present invention. The animal study included injection of traceable agent and sodium nitroprusside (SNP), followed by data acquisition by MRI or fluorescence imaging.

### Materials and Methods

The study included two normal mice which were used in the MRI experiment, and 28 male Sprague Dawley rats (200-250 grams), of which 24 rats were used in the MRI experiment and 4 rats were used in the fluorescence imaging experiment.

### MRI Experiment

In the MRI experiment, the animals were anaesthetized and placed in a specially designed animal MR coil. For the 24 rats (11 treated, 13 control) a 0.5T interventional GE MR system was used and for the mice a 3T clinical MR system was used. The animals were placed inside the MR coil together with a special phantom, containing soap water. Since the phantom contains no living cells, its contrast is generally constant over time. The mean signal of the phantom was later used to normalize the data. A Venflon was fixed in the animal's tail vein prior to placing in the MR system to allow contrast agent injection while the animal is in the MR coil.

T₁-weighted fast spin-echo MR images were acquired in the axial plane. The 0.5T data was acquired with slices of 3 mm, no gap, field-of-view of 14×10.5 cm and a matrix of 256x256. The 3T data was acquired with slices of 1 mm, no gap, field-of view of 10×7.5 cm and a matrix of 256x224.

The following protocol was employed for the mice. A set of MR images were acquired as a baseline set (each image in the set corresponding to a different brain slice). Following baseline acquisition, the mice were intravenously injected with high dose of Gd-DTPA (0.6 ml/kg) MR contrast agent. MR images as described above were acquired repeatedly. One of the mice died about 15 minutes post injection while being scanned by the MRI system. For this mouse, acquisition of MR images continued until about 40 minutes post injection. The other mouse was kept alive throughout the experiment. For this mouse acquisition of MR images continued until about 80 minutes post injection.

The following protocol was employed for the rats. For each rat, a set of MR images were acquired as a baseline set (each image in the set corresponding to a different brain slice). Following baseline acquisition, the rats were intravenously injected with high dose of Gd-DTPA (0.6 ml/kg) MR contrast agent. Subsequently, the rats were subjected to intraperitoneal injection, where treated rats were intraperitoneally injected with 3 mg/kg of Sodium Nitroprusside (SNP) and control rats were intraperitoneally injected with saline. The rats were kept still while being injected and MR images as described above were acquired repeatedly over a period of 40 minutes for each rat, to provide a plurality of sets of MR images.

Once obtained, the MR images were normalized to the average intensity of the phantom in each slice. The entire brain was defined as the region-of-interest (ROI). Normalized intensity maps of the brain were calculated for each set and the color scale was adjusted to depict specific changes. Also calculated for each set was a subtraction map, in which the normalized intensity map of the first set post injection was subtracted from the normalized intensity map of the respective set.

In each set, the normalized intensity of the ROI was averaged and the time-dependence of the average intensity was visualized by plotting the average intensity as a function of the time. A similar procedure was employed for a ROI which was defined in a muscle region of the rat.

### Fluorescence Imaging Experiment

In the fluorescence imaging experiment, the rats were anaesthetized and intravenously injected with sodium fluorescein (4%, 0.5 ml per 200 gr). Subsequently, the rats where subjected to intraperitoneal injection, where 2 treated rats were intraperitoneally injected with 3 mg/kg of SNP and 2 control rats were intraperitoneally injected with saline. 40 minutes following intraperitoneal injection the rats were perfused with Phosphate buffered saline (PBS) for 2 minutes and then with a composition of paraformaldehid (PFA) and PBS (2.5% PFA and 97.5 % PBS) for 10 minutes. The perfusion was performed through the left ventricle of the rat's heart, while the right auricle was cut open and the descending aorta was clamped. The brains were then extracted and placed in PFA 2.5% in PBS. Fluorescence was read using an excitation filter of 465 nm and an emission filter of 540 nm of an IVIS in vivo imaging system (Xenogen Corporation, Alameda, California).

### Results

### MRI Experiment

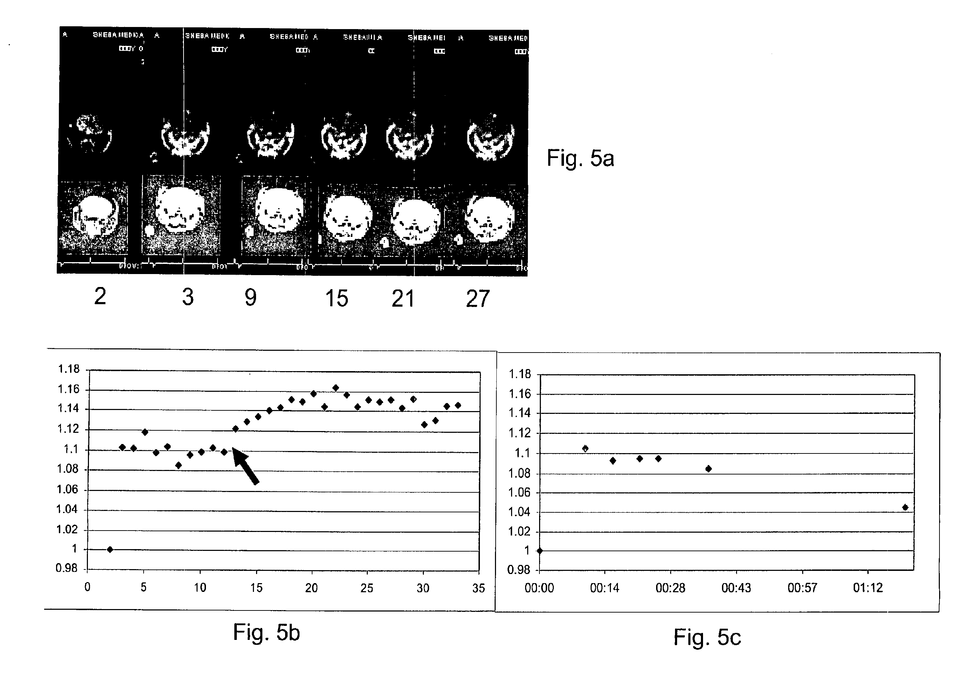

Figures 5a-b show intensity maps (Figure 5a) and an intensity plot (Figure 5b) of the mouse that died during the experiment. Figure 5c shows an intensity plot of the healthy anesthetized mouse which was kept alive throughout the experiment. Shown in Figure 5a are MR images acquired 2, 3, 9, 15, 21 and 27 minutes post injection and normalized intensity maps prepared from the MR images. Shown in Figures 5b-c are the average normalized intensity in dimensionless units as a function of time in minutes. Each point in the intensity plots represents the intensity as averaged over the entire brain. The time instant associated with each point corresponds to the time at which the set was acquired (acquisition initiation). Time of death is marked in Figure 5b by black arrow.

As shown in Figure 5c, the average intensity for the healthy rat decreases substantially monotonically with time, with the highest average intensity at the first time point post injection. The monotonic decrease indicates contrast clearance from the blood system.

As shown in Figure 5b, the average intensity decreases until about 15 minutes post injection, when the rat died. The low intensity at *t* = 2 minutes is the average intensity of the baseline set acquired prior to the injection of contrast agent. Following death, the intensity exhibits a sharp increase as a function of time, reaching a plateau about 10 minutes later. The sharp increase in intensity indicates BBB disruption at death. The plateau is consistent with a situation in which the concentration of contrast agent in the tissue reaches the concentration of the contrast agent in blood. The intensity maps (Figure 5a) show that at the time of death there is a sharp increase in brain tissue enhancement at death while blood pool enhancement remains constant.

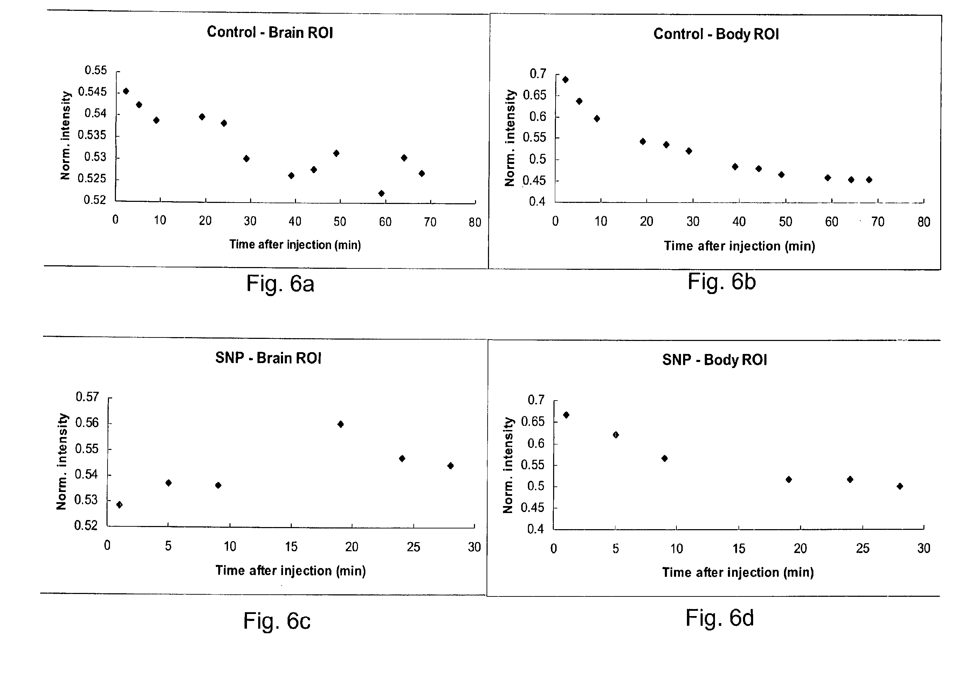

Figures 6a-d show intensity plots (average normalized intensity in dimensionless units as a function of time in minutes) of a control rat (Figure 6a-b) and a rat treated with SNP ((Figure 6c-d). Figures 6a and 6c show intensity plots of the brain ROI and Figures 6b and 6d show intensity plots of the muscle ROI.

As shown in Figures 6b and 6d the intensity in the muscle ROI decreases as a function of time for both rats. This indicates clearance of the contrast agent from the blood. As shown in Figure 6a, the intensity in the brain ROI of the control rat also decreases as a function of time, indicting clearance of the contrast agent from the brain tissue.

As shown in Figure 6c the intensity in the brain of the treated rat increases with time up to the 18th minute post injection. This indicates that the SNP induces BBB disruption resulting in accumulation of contrast agent in the brain.

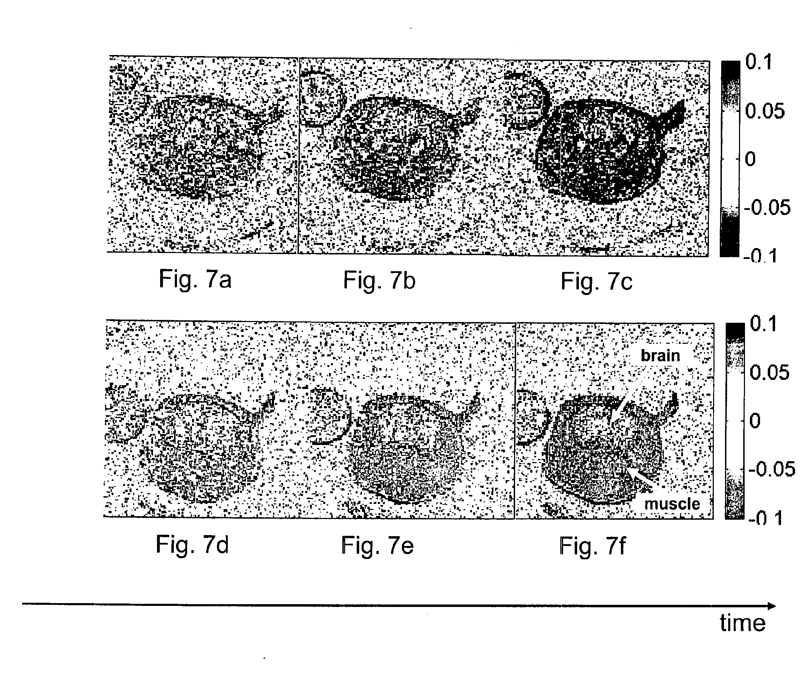

Figures 7a-f are subtraction maps of a treated rat (Figures 7a-c), and a control rat (Figures 7d-f). Shown are subtractions of the first normalized intensity map post injection from the second (Figures 7a and 7d), third (Figures 7b and 7e) and fourth (Figures 7c and 7f) normalized intensity map. Also shown is a color scale wherein, for example, blue color represents a value of about -0.1 and a red color represents a value of about 0.1. The brain tissue and the muscle tissue are marked on Figure 7f The ordinarily skilled person would know how to identify the brain and muscle tissues in Figures 7a-e.

As demonstrated in Figures 7a-f, for both rats there is a decrease in intensity in muscle tissue as a function of time (the color of the ROI is shifted to blue with time). In the treated rat (Figures 7a-c), there is a gradual increase in the intensity of the brain tissue as a function of time (the color of the ROI is shifted to red with time), while in the control rat, there is a gradual decrease in the intensity of the brain tissue (the color of the ROI is shifted to blue with time). This indicates that the SNP induced BBB disruption resulting in accumulation of contrast agent in the brain.

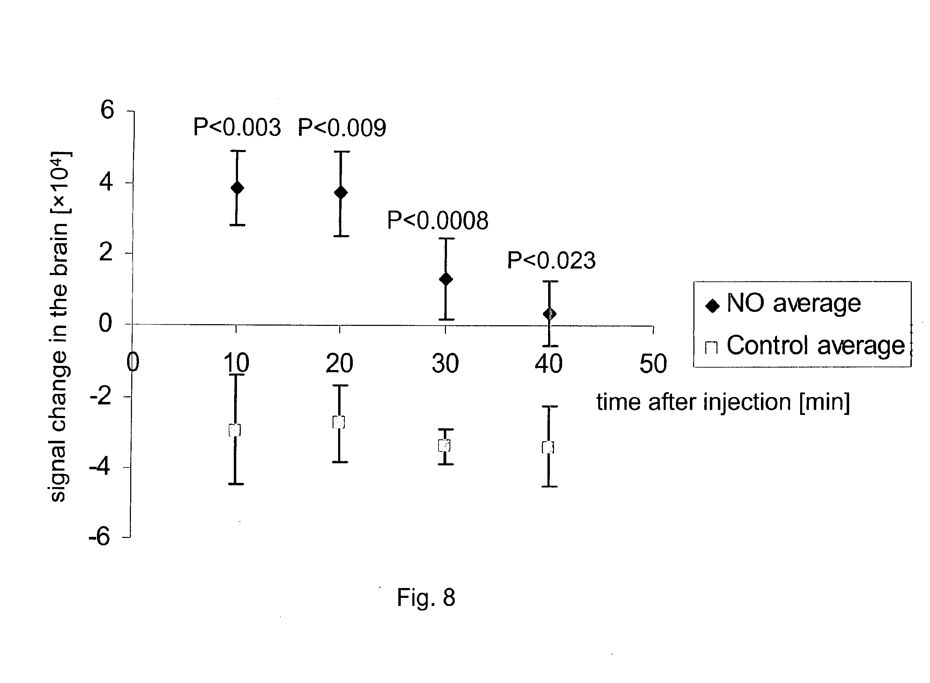

Figure 8 is a graph showing the average subtraction values of the treated rats (blue diamonds) and control rats (pink squares) which were scanned with the 0.5T MRI system. Figure 8 demonstrates that the subtraction values for the treated rats are significantly higher than the subtractions values for the control rats. This indicates that the SNP induces BBB disruption resulting in accumulation of contrast agent in the brain.

### Fluorescence Imaging Experiment

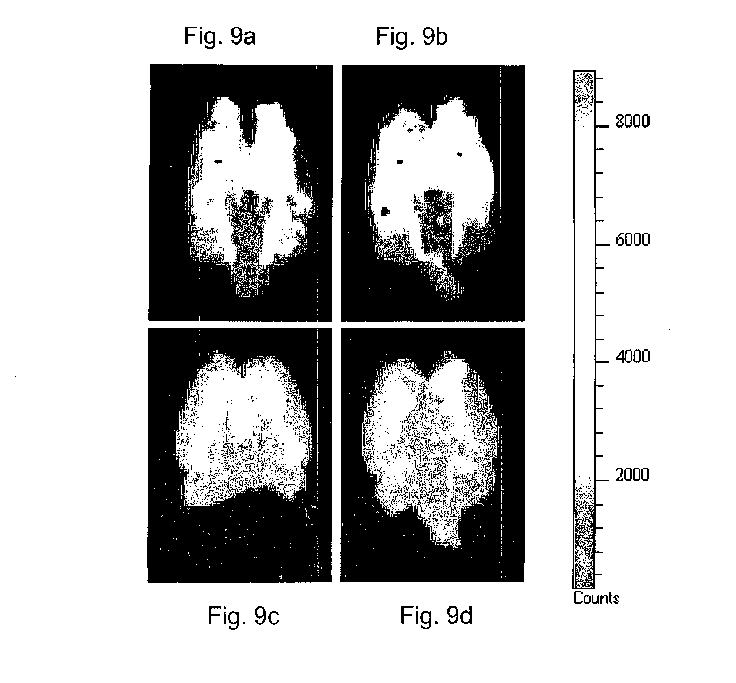

Figures 9a-d are fluorescence images of two treated rats (Figures 9a-b) and two control rats (Figures 9c-d). Shown are brain cuts in the sagittal section. The fluorescence signal is presented in a color code from purple (lowest signal) to red (highest signal). A color scale in units of fluorescence emission counts is shown on the right pane of Figures 9a-d, wherein, for example, purple represents about 400 counts are and red represents about 8800 counts. Figures 9a-b (treated rats) generally exhibit high signal (higher or equal 3000 counts) over most of the sagittal section, with several spots of very high signal (above 6000 counts). Figures 5c-d (control rats) generally exhibit lower signal (lower or equal 2000 counts). This indicates that the SNP induces BBB disruption resulting in accumulation of sodium fluorescein in the brain. In the control rats, BBB reduces entry of sodium fluorescein to the brain.

### EXAMPLE 2

### Human Study

Following is a description of a human study performed according to some embodiments of the present invention. The human study included injection of MRI contrast agent followed by data acquisition by MRI.

### Materials and Methods

The study included 4 volunteers (3 females, 1 male), of which one healthy subject (30-year old male), one schizophrenic subject (19-year old female), one subject suffering from meningioma (43-year old female) and one subject suffering from cappilay angioma (23-year old female).

The volunteers underwent MRI prior to any injection of contrast agent. A special phantom, containing soap water was placed adjacent to the volunteers' head. Subsequently, the volunteers were injected 0.2 ml/kg of Gd-DTPA, followed by a substantially continuous MRI (with soap water phantom) over a period of 40 minutes post injection.

The MRI included repeated acquisition of spin echo (SE) T₁ MR images, to provide a plurality of sets of MR images. All acquisitions were performed using a 3T GE MR system, with slices of 5 mm, gap of 0.5 mm, field-of-view of 26x 19 cm and a matrix of 384x192.

The analysis of MR images according to some embodiments of the present invention was designed to be sensitive to local as well as diffuse BBB abnormalities. In each slice, the data were normalized to the average intensity of the phantom. Intensity maps of the brain as a function of time were then calculated (one map per MR images) using the normalized intensities. The maps were visualized using a color scale which was adjusted to depict specific changes.

The calculated intensity maps were subsequently used for calculating subtraction maps and ratio maps as will now be described.

Each subtraction map corresponded to one set and included subtraction values which were typically obtained by subtracting the normalized intensities of the first set post injection from the normalized intensity map of the respective set. Some subtraction maps included subtraction values which were obtained by subtracting the normalized intensities of the *n*th set from the normalized intensity map of the *m*th set *(m* > *n* > 1). These subtraction maps were useful for assessing late BBB disruption.

Each ratio map corresponded to one set and included ratio values which were typically obtained by dividing the normalized intensities of the respective set by the normalized intensity map of the first set post injection. Some ratio maps included ratio values which were obtained by dividing the normalized intensities of the *m*th set by the normalized intensity map of the nth set (*m* > *n* > 1). These ratio maps were useful for assessing late BBB disruption.

The subtraction maps and ratio maps allowed visualization of the spatial distribution of contrast agent accumulation in the tissue and cerebrospinal fluid (CSF). Broadly speaking, intact BBB (where no increase in accumulation of contrast agent after the first set is expected), can be identified when the subtraction value as manifested by the subtraction maps is negative and/or the ratio value as manifested by the ratio maps is below 1. Conversely, BBB disruption (where accumulation of contrast agent after the first set is expected to increase) can be identified when the subtraction value as manifested by the subtraction maps is positive and/or the ratio value as manifested by the slope maps is above 1.

While the ratio maps are generally noisier than the subtraction maps, they can be more informative in regions in which the original signal is low.

### Results

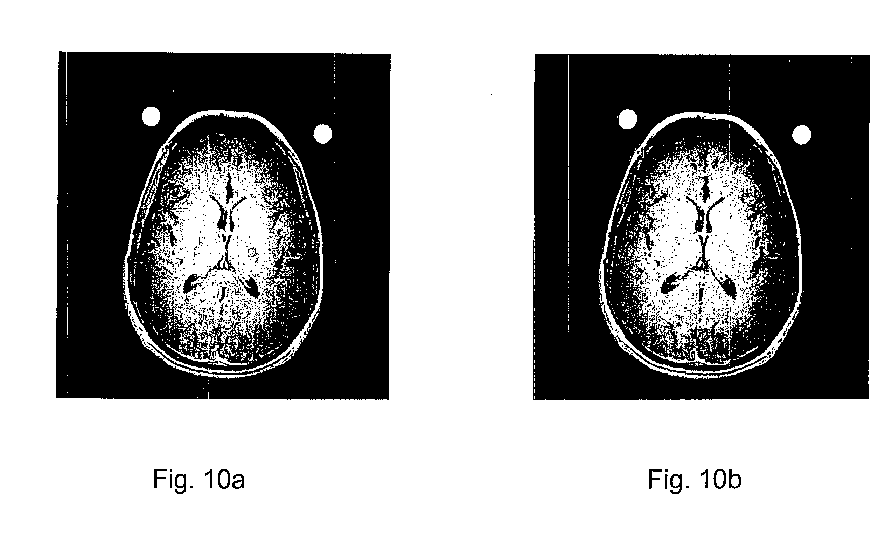

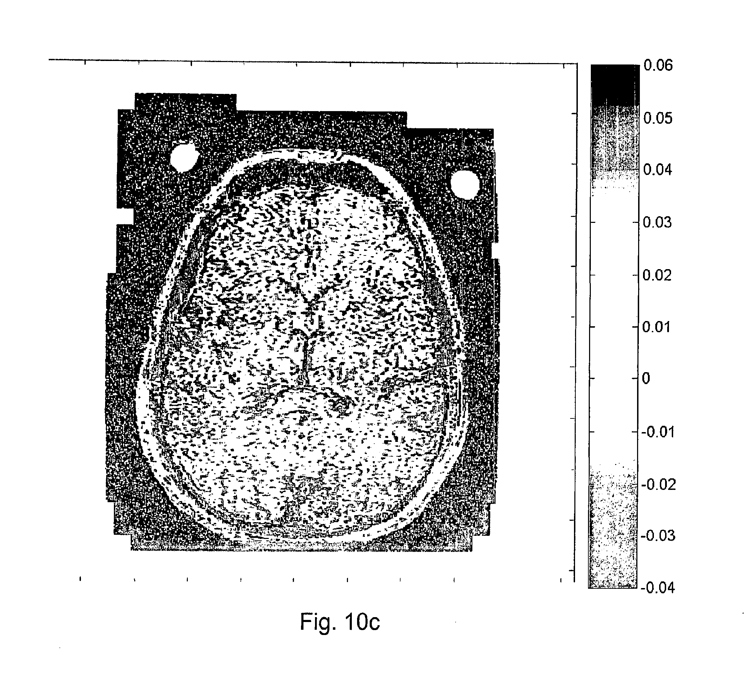

Figures 10a-b are T1-weighted MR images acquired from the healthy subject 1 minute (Figure 10a) and 10 minutes (Figure 10-b) after injection of contrast agent. Figure 10c is a subtraction map corresponding to the MR image shown in Figure 10b. Referring to Figure 10c, the average subtraction value of the tissue is below 1 indicating some clearance of the contrast from the blood system. The subtraction value at the ventricular system is also low, indicating low or no passage of contrast agent thereto. Note that the blood vessels themselves (such as the choroids plexus seen in the lateral ventricles) appear dark blue. This can imply sharp clearance from the blood system. The subtraction map is thus consistent with intact BBB.

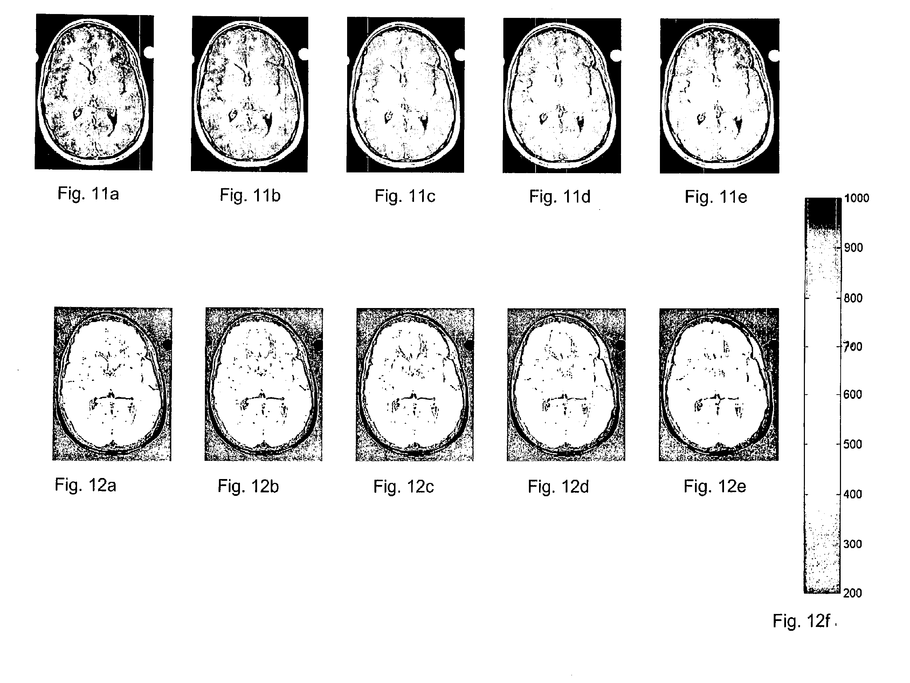

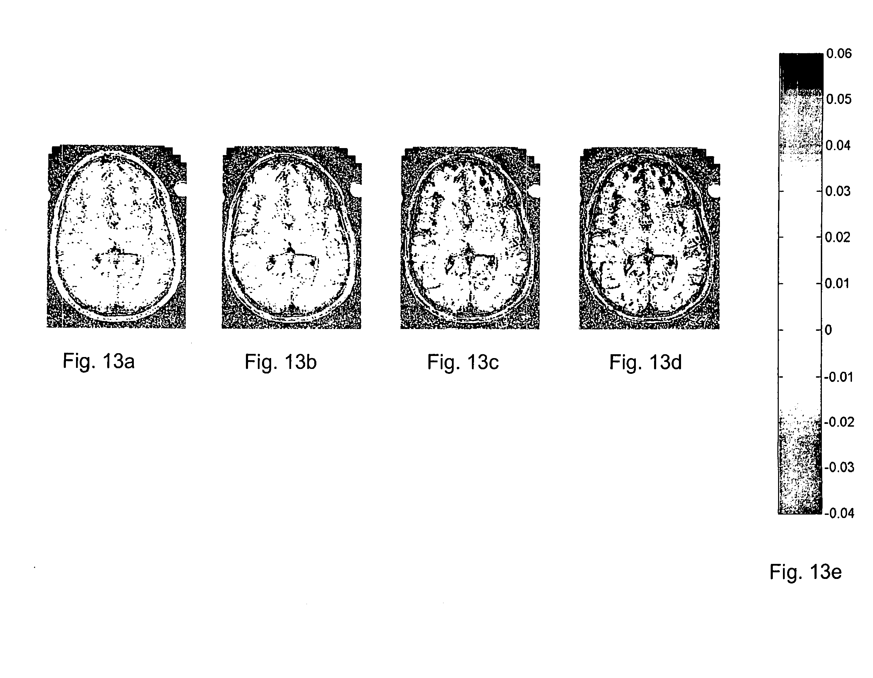

Figures 11a-e are T1-weighted MR images acquired from the schizophrenia patient during acute psychotic state 1, 7, 13, 19 and 23 minutes after injection of contrast agent respectively; Figures 12a-e are intensity maps which respectively correspond to the MR image shown in Figures 11a-e; and Figures 13a-d are the subtraction maps which respectively correspond to the intensity maps shown in Figures 12b-e. The color scale for Figures 12a-e is shown in Figure 12f, and the color scale for Figures 13a-d is shown in Figure 13e.

The overall subtraction value is slightly above 0 in Figure 13a, and decreases to values below 0 with time. The enhancement in the ventricles increases with time, indicating BBB disruption. Note that the blood vessels such as the choroids plexus seen in the lateral ventricles appear dark blue. As stated, this can be explained by sharp clearance from the blood system.

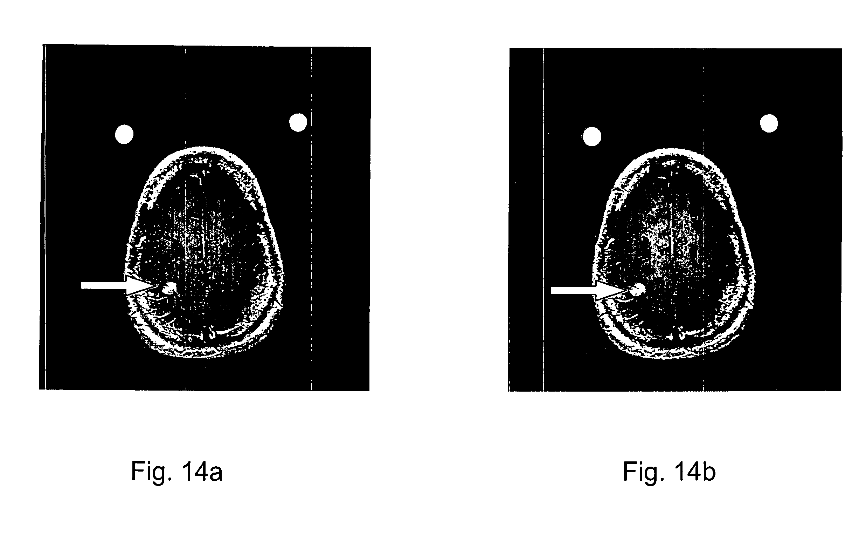

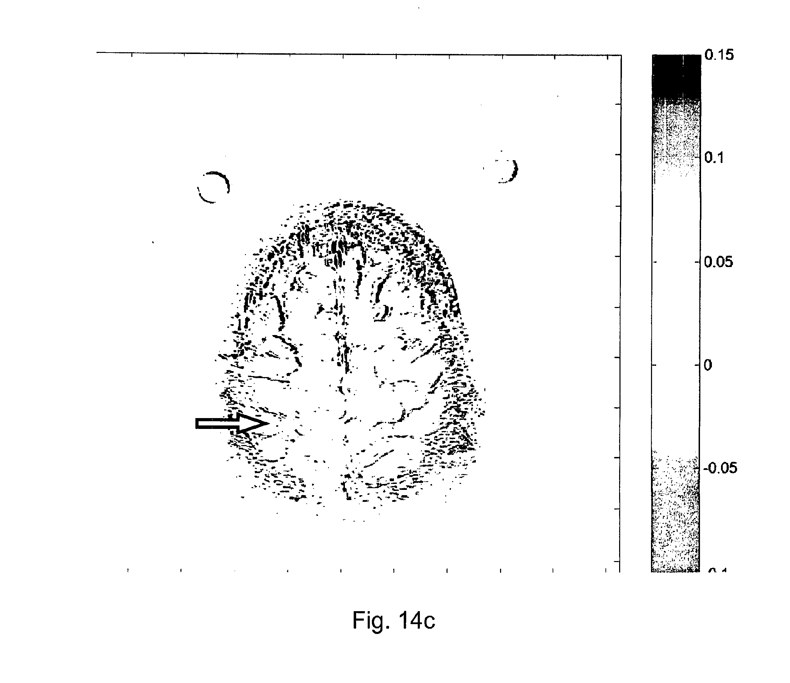

Figures 14a-b are T1-weighted MR images acquired from a subject suffering from meningioma 1 minute (Figure 14a) and 7 minutes (Figure 14b) after injection of contrast agent. Figure 14c is a subtraction map corresponding to the MR image shown in Figure 14b. The tumor is marked in Figures 14a-c by an arrow. Referring to Figure 14c, the overall subtraction value of the tissue is close to 0 indicating minimal contrast clearance from the blood system but no accumulation of contrast in the tissue. This subtraction map is thus consistent with a globally intact BBB. Yet, the region of the tumor appears enhanced in the subtraction map, consistent with local BBB disruption.

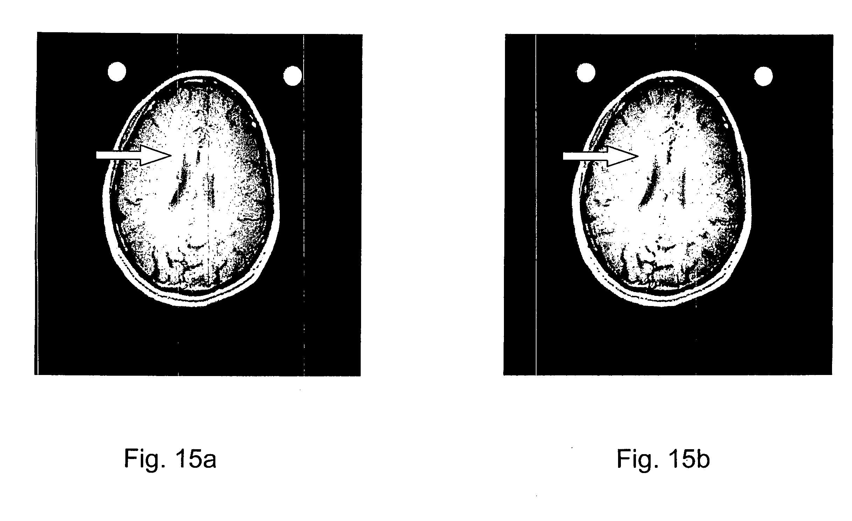

Figures 15a-b are T1-weighted MR images acquired from a subject suffering from capillary angioma, who was treated with Hexakapron 2 gr/day due to heavy menses. Figure 15a was acquired 1 minute after injection of the contrast agent and Figure 15b was acquired and 10 minutes after injection the of contrast agent.

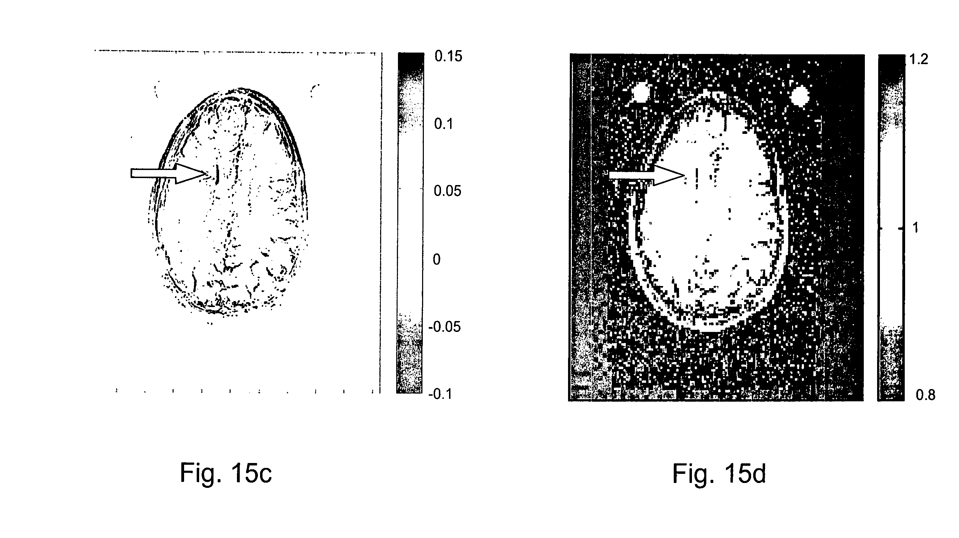

Figure 15c is a subtraction map corresponding to the MR image shown in Figure 15b, and Figure 15d is a ratio map corresponding to the MR image shown in Figure 15b.

Capillary angioma is a vascular malformation manifested as a network of aneurysmally dilated capillaries. The angioma is marked in Figures 15a-d by an arrow. In the MR images (Figures 15a-b) the angioma is depicted as an enhanced region, similarly to other tumors. In the subtraction map (Figure 15c) and slope map (Figure 15d) the angioma appears dark blue (subtraction value of below -0.1, and ratio value of below 0.8) due to the sharp decrease in the blood signal (these values are saturated). This is consistent with intact blood vessels and not with tumors that are accompanied by abnormal BBB (such as the tumor shown in Figure 14c).

Although the invention has been described in conjunction with specific embodiments thereof, it is evident that many alternatives, modifications and variations will be apparent to those skilled in the art. Accordingly, it is intended to embrace all such alternatives, modifications and variations that fall within the spirit and broad scope of the appended claims.

All publications, patents and patent applications mentioned in this specification are herein incorporated in their entirety by reference into the specification, to the same extent as if each individual publication, patent or patent application was specifically and individually indicated to be incorporated herein by reference. In addition, citation or identification of any reference in this application shall not be construed as an admission that such reference is available as prior art to the present invention. To the extent that section headings are used, they should not be construed as necessarily limiting.

## Claims

1. A system for disrupting blood-brain barrier of a subject, comprising a transcranial magnetic stimulation device having a mode of operation which effects a blood-brain barrier disruption.

2. The system of claim 1, further comprising a magnetic resonance imaging system for generating a plurality of magnetic resonance images of the subject's brain acquired over a predetermined time-period, and apparatus for assessing blood-brain barrier function based on the magnetic resonance images and for issuing a report regarding the blood-brain barrier function.

3. The system of claim 2, wherein said apparatus comprises:
an intensity map constructor for constructing, for each magnetic resonance image, an intensity map;
a variation map constructor for constructing at least one variation map describing variations in concentration of an MRI contrast agent in said brain by detecting dissimilarities among a pair of intensity maps; and
blood-brain barrier function assessment unit configured for assessing the blood-brain barrier function based on said variations.

4. The system of claim 3, wherein said assessment unit is configured for assigning a representative intensity value for each magnetic resonance image and determining a time-dependence of said representative intensity value.

5. The system of claim 4, wherein said assessment unit is configured for generating a graph describing said time-dependence.

6. The system according to any of claims 4 and 5, wherein each representative intensity value is assigned by averaging intensities over a respective magnetic resonance image.

7. The system according to any of claims 3-6, wherein each magnetic resonance image comprises a sliced magnetic resonance image, and wherein said comparison is performed slice by slice.

8. The system according to any of claims 3-7, wherein said at least one variation map comprises at least one map selected from the group consisting of a subtraction map, a slope map and a ratio map.

9. The system according to any of claims claim 2-8, wherein said apparatus is configured for assessing blood-brain barrier function locally.

10. The system according to any of claims 2-8, wherein said apparatus is configured for assessing blood-brain barrier function globally.

11. A method of monitoring delivery of a compound to the brain of the subject following stimulation by transcranial magnetic stimulation device to temporarily effect a blood-brain barrier disruption and administering of the compound to the subject, the method comprising acquiring a plurality of magnetic resonance images of the subject's brain over a predetermined time-period, and monitoring said delivery using said images.

12. The method of claim 11, further comprising assessing blood-brain barrier function based on said images.

13. The method of claim 12, wherein said assessment of said blood-brain barrier function is done locally.

14. The method of claim 12, wherein said assessment of said blood-brain barrier function is done globally.

15. The method according to any of claims 12-14, wherein said assessing blood-brain barrier function comprises comparing at least two of said plurality of magnetic resonance images thereamongst so as to determine variations in concentration of an MRI contrast agent in said brain, wherein said blood-brain barrier function is assessed based on said variations.
